# EUROPEAN PATENT SPECIFICATION

(11) **EP 1 904 641 B1**
(45) Date of publication and mention of the grant of the patent: **16.09.2009**
(21) Application number: 06777777.1
(22) Date of filing: 14.07.2006
(51) Int. Cl.: C12P 21/04

(54) **PROCESS FOR THE MANUFACTURE OF EPTIFIBATIDE**
VERFAHREN ZUR HERSTELLUNG VON EPTIFIBATID
PROCEDE DE FABRICATION D'EPTIFIBATIDE

(30) Priority: 15.07.2005 US 699818 P
(43) Date of publication of application: 02.04.2008
(73) Proprietor: Solvay SA, 1050 Brussels (BE)
(72) Inventor: COLLIN, André, B-1081 Brussels (BE); TAMBUYSER, Thierry, B-1120 Brussels (BE)
(74) Representative: Mross, Stefan P.M.
(86) International application number: PCT/EP2006/064253
(87) International publication number: WO 2007/009946

(56) References cited:
- WO-A-02/072132
- WO-A-03/093302
- WO-A-20/05100381
- WO-A-20/06041945
- US-A- 5 686 571
- US-A- 5 795 868
- "MILLENIUM PHARMACEUTICALS INC URL - www.fda.gov/medwatch/SAFETY/2004/julPI/Int eegrilin_PI.pdf" INTERNET ARTICLE, [Online] April 2004 (2004-04), XP002304465 Retrieved from the Internet: URL:www.fda.gov/medwatch/SAFETY/2004/julPI /Inteegrilin_PI.pdf> [retrieved on 2004-08-11]
- ANONYMOUS: "INTEGRILIN SOLUTION FOR INJECTION" INTERNET ARTICLE, [Online] 27 May 2005 (2005-05-27), XP002400963 Retrieved from the Internet: URL:http://www.medsafe.govt.nz/Profs/datas heet/I/Integrilininj.htm> [retrieved on 2006-09-27]
- SCARBOROUGH R M ET AL: "DESIGN OF POTENT AND SPECIFIC INTEGRIN ANTAGONISTS. ÖPEPTIDE ANTAGONISTS WITH HIGH SPECIFICITY FOR GLYCOPROTEIN IIB-IIIA" JOURNAL OF BIOLOGICAL CHEMISTRY, AMERICAN SOCIETY OF BIOLOCHEMICAL BIOLOGISTS, BIRMINGHAM,, US, vol. 268, no. 2, 15 January 1993 (1993-01-15), pages 1066-1073, XP000336707 ISSN: 0021-9258
- PHILLIPS DAVID R ET AL: "Clinical pharmacology of eptifibatide" AMERICAN JOURNAL OF CARDIOLOGY, vol. 80, no. 4A, 1997, pages 11B-20B, XP009072969 ISSN: 0002-9149
- ANONYMOUS: "INTEGRILIN" INTERNET ARTICLE, [Online] XP002400964 Retrieved from the Internet: URL:http://www.millennium.com/products/int egrilin/index.asp> [retrieved on 2006-09-27]

## Description

The present invention relates to a process for the manufacture of concentrated eptifibatide acetate material, in particular to such a process comprising a purification step.

Eptifibatide, which is commercialised by MILLENNIUM PHARMACEUTICALS under the name Integrelin^{™} is a cyclical heptapeptide of sequence 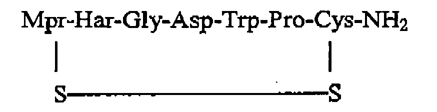 that selectively blocks the platelet glycoprotein IIb/ IIIa receptor. It reversibly binds to platelets and has a short half-life. It has demonstrated efficacy as an intravenous solution in the treatment of patients during coronary angioplasty, myocardial infarction and angina.

Callens discloses in the IBC talk, San Diego, 1999 a method of manufacturing eptifibatide.

Scarborough et al. US 5,686,571 discloses laboratory-scale chromatography of eptifibatide by reversed phase HPLC using a gradient of acetonitrile in trifluoroacetic acid, wherein the chromatography fractions are directly lyophilized.

It was desirable to have available a process which allows for manufacture of eptifibatide having required purity for its applications and which presents advantages in terms of productivity and required manufacturing equipment.

In consequence, the invention concerns a process for manufacturing a concentrated cyclic heptapeptide eptifibatide acetate material which comprises subjecting a solution of the cyclic heptapeptide eptifibatide acetate in a solvent comprising water and acetonitrile to an evaporation step comprising evaporating a mixture of water and acetonitrile and further comprising adding water to the solution during the evaporation step so as to reduce the acetonitrile content in the concentration solution to less than or equal to 20 mg/kg which evaporation step is carried out at a temperature from 0°C to less than or equal to 40°C and, wherein the pH of said solution is from 3 to 5.

It has been found, surprisingly, that the process according to the invention allows to provide concentrated pure eptifibatide acetate without substantial formation of by-products, in particular dimerization of eptifibatide, during the concentration step. It also allows in particular to meet specifications concerning purification related organic impurities such as for example organic solvents, in particular acetonitrile.

In the present invention, "solution of eptifibatide acetate" is intended to denote a substantially homogeneous, preferably a totally homogeneous liquid phase containing eptifibatide acetate dissolved therein.

In the following description of the present invention, "eptifibatide derivative" is intended to denote the acetate form of the cyclic heptapeptide. The eptifibatide acetate is the commercialized drug.

In the process according to the invention the pH of the solution is less than or equal to 5 and more preferably less than or equal to about 4.6. In the process according to the invention, the pH of the solution is more than or equal to 3 and preferably more than or equal to 4.

In the process according to the invention the pH of the solution is Preferably maintained at the values indicated above throughout the concentration step. It is not preferred to work outside the indicated values though it might be possible to do so for a short period of time, without materially affecting the results obtained by virtue of the teaching of the present invention.

In the process according to the invention the pH of the solution is preferably maintained within a given interval around a chosen target pH value, as described above. Preferably, this interval is about ±0.5. The pH is generally maintained by measuring and adjusting, if necessary, by addition of pH controlling agent. A preferred pH controlling agent is an acetate buffer, for example in particular a mixture of eptifibatide acetate and suitably controlled amount of acetic acid. The pH controlling agent can be added to the solution before or during the concentration step.

In the process according to the invention, the concentration step is carried out at a temperature less than or equal to 40°C preferably less than or equal to 35°C and more preferably less than or equal to 30°C. In the process according to the invention, the concentration step is carried out at a temperature higher than or equal to 0°C and more preferably higher than or equal to 20°C. A concentration step carried out at a temperature of about 30°C is more particularly preferred.

In the process according to the invention, the temperature is preferably maintained at the values indicated above throughout the concentration step. It is not preferred to work outside the indicated values though it might be possible to do so for a short period of time, without materially affecting the results obtained by virtue of the teaching of the present invention.

In the process according to the invention the temperature during the concentration step is preferably maintained within a given interval around a chosen target temperature value, as described above. Preferably, this interval is about ±5°C and more preferably ±2°C.

In the process according to the invention the solvent comprises water and acetonitrile.

In this embodiment the volume ratio of water to acetonitrile is generally equal to or higher than 50:50 relative to the mixture water/acetonitrile. Preferably this ratio is equal to or higher than 70:30. In this embodiment, the volume ratio of water to acetonitrile is generally equal to or lower than 90:10 relative to the mixture water/acetonitrile, Preferably this ratio is equal to or lower than about 80:20.

In this process according to the invention, the solvent preferably comprises acetic acid. When the solvent contains water and acetonitrile, the acetic acid content is preferably 0.1-1 volume parts per 100 volume parts of water/acetonitrile mixture and more preferably about 0.3 volume parts per 100 volume parts of water/acetonitrile mixture.

In the process according to the invention, the eptifibatide derivative contained in the solution consists essentially of eptifibatide acetate.

In the process according to the invention, the purity of the eptifibatide derivative in the solution subjected to the concentration operation is generally at equal to or higher than 98.5 area % determined by HPLC. Preferably this purity is equal to or higher than 99 area % and more preferably equal to or higher than 99.5 area %. Generally, the purity of the eptifibatide derivative in the solution subjected to the concentration operation does not exceed 99.7 area % determined by HPLC.

In the process according to the invention, the eptifibatide derivative concentration in the solution subjected to the concentration operation is generally equal to or higher than 0.5 wt. %, preferably equal to or higher than 1 wt. % and more preferably equal to or higher than 2 wt. %. In the process according to the invention, the eptifibatide derivative concentration in the solution subjected to the concentration operation is generally lower than 4 wt. %, preferably equal to or lower than 3 wt. %.

The material recovered from the evaporation operation is a concentrated solution of eptifibatide derivative.

The concentrated eptifibatide derivative solution recovered from the evaporation operation has preferably an eptifibatide derivative concentration below the solubility limit of the eptifibatide derivative in the solvent having been subjected to evaporation, at the temperature of the concentration step.
Preferably, the solution has a concentration of at least 70 % molar of the solubility limit of the eptifibatide derivative in the solvent having been subjected to evaporation, at the temperature of the concentration step, more preferably at least 75 % molar and most preferably about 80 % molar of the solubility limit. It generally does not exceed about 85 % molar of the solubility limit. It has been found that limiting the evaporation step to this value allows for improved control of the process, thus avoiding undesired precipitation and associated loss of product lots. In a less advantageous embodiment, the eptifibatide derivative concentration is substantially below that solubility limit.

In the process according to the invention, wherein a mixture of water and acetonitrile is used, the concentrated eptifibatide derivative solution has generally an eptifibatide derivative concentration equal to or lower than the about 5 wt. % but preferably equal to or higher than 4 wt. %.

In the process according to the invention, the purity of the eptifibatide derivative in the concentrated material recovered from the evaporation operation is generally at equal to or higher than 98.5 area % determined by HPLC. Preferably this purity is equal to or higher than 99 area % and more preferably equal to or higher than 99.5 area %. Generally, the purity of the eptifibatide derivative in the concentrated material recovered from the evaporation operation does not exceed 99.7 area % determined by HPLC.

In the process according to the invention, the content of dimeric eptifibatide derivatives in the concentrated material recovered from the concentration operation is generally equal to or lower than 0.2 area % determined by HPLC, preferably equal to or lower than 0.1 area %. If dimeric eptifibatide derivatives are present in the concentrated material recovered from the concentration operation, their concentration can be at least 0.05 area % determined by HPLC.

The process according to the invention, comprises evaporating a mixture of water and acetonitrile from the solution. The mixture is generally evaporated at a pressure higher than or equal to 0.001 bars, preferably higher than or equal to 0.01 bars. The solvent is generally evaporated at a pressure lower than or equal to 1 bar, preferably lower than or equal to 0.5 bars, more preferably lower than or equal to 0.1 bars.

By evaporating a mixture of water and acetonitrile, the concentration of acetonitrile in the solution is reduced.

In the process according to the invention, water is added to the solution during the evaporation step so as to further reduce the content and substantially eliminate acetonitrile in the concentrated solution. Acetonitrile content in the concentrated solution is less or equal than about 20 mg/kg.

In a preferred embodiment, the process according to the invention comprises steps (a) to (d) wherein
(a) crude eptifibatide derivative is manufactured by coupling of peptide fragments, for example according to the reaction sequence disclosed in Callens, IBC Talk cited above;
(b) the crude eptifibatide derivative is subjected to a purification operation comprising at least one liquid chromatography operation to obtain a solution of eptifibatide derivative comprising water and acetonitrile;
(c) the solution of eptifibatide derivative is subjected to the evaporation, step according the invention to obtain material having increased concentration of eptifibatide; and
(d) the material having increased concentration of eptifibatide is subjected to a lyophilization operation to obtain eptifibatide.

The example here after is intended to illustrate the invention without however limiting its scope.

### Example

801 of a solution of eptifibatide acetate having a purity of 99.5 % measured by analytical HPLC on a C-18 reversed phase column, having an eptifibatide concentration of 15.2 g/l in a mixture water/acetonitrile/acetic acid 70 parts by volume/30 parts by volume/0.3 parts by volume said solution having a pH of 4.6. was introduced into a tubular climbing film evaporator and an acetonitrile/water mixture was evaporated at a temperature kept at about 30°C. During the evaporation, 301 of water were fed continuously at the rate of evaporation. The evaporation was then continued until the volume of the solution had decreased to about 30 1. The pH of this solution was 4.2. A concentrated solution of eptifibatide acetate having a concentration of 40.5 g/l a and purity of 99.5 % and no detectable dimer was recovered and introduced into a lyophilization step. Eptifibatide acetate having appropriate purity for use in medicament was obtained in high yield from that step.

The process according to the invention allows to reduce the volume of solution introduced into further isolation procedures such as cost-intensive lyophilization step while maintaining required purity of eptifibatide derivative for pharmaceutical application and substantially avoiding formation of by-products, in particular dimeric by-products.

## Claims

1. A process for manufacturing a concentrated cyclic heptapeptide eptifibatide acetate material which comprises subjecting a solution of the cyclic heptapeptide eptifibatide acetate in a solvent comprising water and acetonitrile to an evaporation step comprising evaporating a mixture of water and acetonitrile, and further comprising adding water to the solution during the evaporation step so as to reduce the acetonitrile content in the concentrated solution to less than or equal to 20 mg/kg, which evaporation step is carried out at a temperature from 0°C to less than or equal to 40°C and wherein the pH of said solution is from 3 to 5.

2. The process according to claim 1, wherein the temperature is from 0 to 35°C.

3. The process according to claim 2, wherein the temperature is from 20 to 35°C,

4. The process according to anyone of claims 1 to 3, wherein the solvent is evaporated at a pressure from 0.001 to 0.5 bars.

5. The process according to many one of claims 1 to 4 wherein a concentrated solution of eptifibatide acetate is recovered from the evaporation which has a concentration of eptifibatide acetate which is from 70 % molar to 85 % molar of the solubility limit of the eptifibatide acetate in the solvent having been subjected to evaporation at the temperature of the evaporation step.

6. The process according to anyone of claims 1 to 5, wherein the solvent comprises acetic acid.

7. The process according to anyone of claims 1 to 6, wherein the eptifibatide acetate concentration in the solution subjected to the concentration operation is from 0.5 to 3 wt. %.

8. The process according to anyone of claims 1 to 7, wherein the material recovered from the evaporation is a concentrated solution of eptifibatide acetate.

9. The process according to claim 8, wherein the concentrated solution has an eptifibatide salt concentration of from 4 to 5 wt. %.

10. The process according to anyone of claims 1 to 9, wherein
(a) the solution of eptifibatide acetate is obtained by a purification operation comprising at least one liquid chromatography operation;
(b) the solution of eptifibatide acetate is subjected to the evaporation step according to anyone of claims 1 to 10 to obtain material having increased concentration of eptifibatide acetate; and
(c) the material having increased concentration of eptifibatide acetate is subjected to a lyophilization operation to obtain the cyclic heptapeptide eptifibatide acetate.

## Patentansprüche

1. Verfahren zur Herstellung eines konzentrierten cyclischen Heptapeptid-Eptifibatidacetat-Materials, welches umfasst dass man eine Lösung des cyclischen Heptapeptid-Eptifibatidacetats in einem Lösungsmittel, welches Wasser und Acetonitril umfasst, einem Verdampfungsschritt unterwirft welches das Verdampfen einer Mischung von Wasser und Acetonitril und ferner das Hinzusetzen von Wasser zu der Lösung während des Verdampfungsschrittes umfasst, wodurch der Acetonitrilgehalt in der konzentrierten Lösung auf weniger als oder gleich 20 mg/kg verringert wird, wobei der Verdampfungsschritt bei einer Temperatur von 0°C bis weniger als oder gleich 40°C durchgeführt wird und wobei der pH-Wert der Lösung von 3 bis 5 beträgt.

2. Verfahren gemäß Anspruch 1, wobei die Temperatur von 0 bis 35°C beträgt.

3. Verfahren gemäß Anspruch 2, wobei die Temperatur von 20 bis 35°C beträgt.

4. Verfahren gemäß einem beliebigen der Ansprüche 1 bis 3, wobei das Lösungsmittel bei einem Druck von 0,001 bis 0,5 bar abgedampft wird.

5. Verfahren gemäß einem beliebigen der Ansprüche 1 bis 4, wobei eine konzentrierte Lösung von Eptifibatidacetat aus der Verdampfung gewonnen wird, welche eine Konzentration an Eptifibatidacetat aufweist, welche zwischen 70 % molar bis 85 % molar der Löslichkeitsgrenze des Eptifibatidacetats in der Lösung liegt, welche einer Verdampfung bei der Temperatur des Verdampfungsschrittes unterzogen worden ist.

6. Verfahren gemäß einem beliebigen der Ansprüche 1 bis 5, wobei das Lösungsmittel Essigsäure umfasst.

7. Verfahren gemäß einem beliebigen der Ansprüche 1 bis 6, wobei die Eptifibatidacetatkonzentration in der Lösung, die dem Konzentrierungsvorgang unterzogen wird, von 0,5 bis 3 Gew.-% beträgt.

8. Verfahren gemäß einem beliebigen der Ansprüche 1 bis 7, wobei das Material, welches von der Verdampfung rückgewonnen wurde, eine konzentrierte Lösung von Eptifibatidacetat ist.

9. Verfahren gemäß Anspruch 8, wobei die konzentrierte Lösung eine Eptifibatidsalzkonzentration von 4 bis 5 Gew.-% aufweist.

10. Verfahren gemäß einem beliebigen der Ansprüche 1 bis 9, wobei
(a) die Lösung von Eptifibatidacetat durch einen Reinigungsvorgang erhalten wird, der mindestens einen Flüssigkeitschromatografievorgang umfasst;
(b) die Lösung von Eptifibatidacetat dem Verdampfungsschritt gemäß einem beliebigen der Ansprüche 1 bis 10 ausgesetzt wird, um ein Material zu erhalten, welches eine erhöhte Konzentration an Eptifibatidacetat aufweist; und
(c) das Material mit erhöhter Konzentration an Eptifibatidacetat einem Lyophilisationsvorgang unterzogen wird, um das cyclische Heptapeptid-Eptifibatidacetat zu erhalten.

## Revendications

1. Procédé de fabrication d'une matière d'heptapeptide cyclique acétate d'eptifibatide concentrée, **caractérisé en ce qu'**il comprend l'étape consistant à soumettre une solution de l'heptapeptide cyclique acétate d'eptifibatide dans un solvant comprenant de l'eau et de l'acétonitrile à une étape d'évaporation comprenant l'évaporation d'un mélange d'eau et d'acétonitrile, et comprenant en outre l'addition d'eau à la solution pendant l'étape d'évaporation de manière à réduire la teneur en acétonitrile dans la solution concentrée à une valeur inférieure ou égale à 20 mg/kg, laquelle étape d'évaporation est effectuée à une température de 0°C à une valeur inférieure ou égale à 40°C, le pH de ladite solution étant de 3 à 5.

2. Procédé selon la revendication 1, **caractérisé en ce que** la température est de 0 à 35°C.

3. Procédé selon la revendication 2, **caractérisé en ce que** la température est de 20 à 35°C.

4. Procédé selon l'une quelconque des revendications 1 à 3, **caractérisé en ce que** le solvant est évaporé à une pression de 0,001 à 0,5 bar.

5. Procédé selon l'une quelconque des revendications 1 à 4, **caractérisé en ce qu'**une solution concentrée d'acétate d'eptifibatide est récupérée de l'évaporation qui a une concentration d'acétate d'eptifibatide qui est de 70 % molaire à 85 % molaire de la limite de solubilité de l'acétate d'eptifibatide dans le solvant ayant été soumis à l'évaporation à la température de l'étape d'évaporation.

6. Procédé selon l'une quelconque des revendications 1 à 5, **caractérisé en ce que** le solvant comprend de l'acide acétique.

7. Procédé selon l'une quelconque des revendications 1 à 6, **caractérisé en ce que** la concentration d'acétate d'eptifibatide dans la solution soumise à l'opération de concentration est de 0,5 à 3 % en poids.

8. Procédé selon l'une quelconque des revendications 1 à 7, **caractérisé en ce que** la matière récupérée de l'évaporation est une solution concentrée d'acétate d'eptifibatide.

9. Procédé selon la revendication 8, **caractérisé en ce que** la solution concentrée a une concentration de sel d'eptifibatide de 4 à 5 % en poids.

10. Procédé selon l'une quelconque des revendications 1 à 9, **caractérisé en ce que**
(a) la solution d'acétate d'eptifibatide est obtenue par une opération de purification comprenant au moins une opération de chromatographie en phase liquide ;
(b) la solution d'acétate d'eptifibatide est soumise à l'étape d'évaporation selon l'une quelconque des revendications 1 à 10 pour obtenir une matière ayant une concentration accrue d'acétate d'eptifibatide et
(c) la matière ayant une concentration accrue d'acétate d'eptifibatide est soumise à une opération de lyophilization pour obtenir l'heptapeptide cyclique acétate d'eptifibatide.
